## Europäisches Patentamt
## European Patent Office
## Office européen des brevets

(19)

(11) Publication number: **0 009 545**
**B1**

(12) **EUROPEAN PATENT SPECIFICATION**

(45) Date of publication of patent specification: **08.12.82**

(51) Int. Cl.³: **C 07 C 57/04,**
C 07 C 51/42, B 01 D 5/00

(21) Application number: **79102324.5**

(22) Date of filing: **09.07.79**

(54) Acrylic acid recovery with recycle quench.

(30) Priority: **13.09.78 US 942090**

(43) Date of publication of application:
**16.04.80 Bulletin 80/8**

(45) Publication of the grant of the patent:
**08.12.82 Bulletin 82/49**

(84) Designated Contracting States:
**BE CH DE FR GB IT NL**

(56) References cited:
**GB - A - 1 293 848**
**GB - A - 1 466 209**
**US - A - 3 717 675**

(73) Proprietor: **THE STANDARD OIL COMPANY**
**Midland Building**
**Cleveland, Ohio 44115 (US)**

(72) Inventor: **Wagner, David Rudolph**
**3613 W. 120 St.**
**Cleveland, Ohio 44111 (US)**

(74) Representative: **Redies, Bernd, Dr. rer. nat. et al,**
**Redies, Redies, Türk & Gille Brucknerstrasse 20**
**D-4000 Düsseldorf 13 (DE)**

Courier Press, Leamington Spa, England.

Acrylic acid recovery with recycle quench

## Background of the Invention

Acrylic acid is produced by the catalytic vapor phase oxidation of propylene or acrolein with molecular oxygen. Acrylic acid may be formed directly from acrolein using catalyst such as found in U.S. 3,736,354 and 3,644,509. Acrylic acid may also be formed from propylene in a one or two-stage operation. Although directed to the production of esters, U.S. 4,060,545 discloses catalyst that are effective to convert propylene directly to acrylic acid or to convert propylene to acrolein which is then reacted to acrylic acid. The catalyst used in these processes are known as activated oxide complexes.

The temperature of the reaction is typically between 300—600°C. The ratio of reactants may vary widely depending on whether propylene or acrolein is used as feed. Steam can be added to the reactor to increase selectivity of the catalyst. The reaction produces a gaseous reactor effluent containing acrylic acid, acrolein, water and various impurities. These impurities consist of such components as acetic acid and inert gases.

In order to effectively recover acrylic acid, it is first necessary to obtain the acrylic acid in an aqueous solution suitable for later purification. The prior art has attempted this in a variety of ways. As described in the prior art section of US—PS 3,926,744, the gaseous reactor effluent is first cooled by using a liquid which is obtained by recycling cooling constituents of the condensate from this first step as a quenching liquid. A similar processes and equipment are known from GB—PS 1,466,209 and GB—PS 1,293,848. These processes have several disadvantages in that polymers deposit in the equipment, and the equipment size must be relatively large to handle both the quench liquid and the aqueous solution of acrylic acid.

US—PS 3,926,744 presents another solution to the formation of an aqueous acrylic acid solution by utilizing organic materials such as hexyl alcohol in the quench system to decrease the formation of polymers and aid in the separation of acrolein.

US—PS 3,717,675 discloses a process wherein the reaction gas is first indirectly cooled to a temperature of 100—200°C, and the precooled gases are then scrubbed with water to a temperature between 30° and 90°C. This process suffers from the disadvantage of requiring an extremely large indirect heat exchanger to handle the total gaseous reactor effluent.

The present invention reduces the size of equipment involved, and improves the recovery of acrylic acid from the gaseous reactor effluent by means of a unique quench recycle.

## Summary of the Invention

It has been discovered that the recovery of acrylic acid can be improved in the process for the recovery of acrylic acid from a gaseous reactor effluent containing acrylic acid, acrolein, water and impurities produced from the catalytic vapor phase oxidation of propylene or acrolein comprising the steps of:

a) quenching the gaseous reactor effluent with a quench liquid wherein a first liquid stream containing acrylic acid and a first vapor stream containing acrylic acid are formed;

b) indirectly cooling said first vapor stream to form a second liquid stream containing acrylic acid and a second vapor stream;

c) passing said second liquid stream as the quench liquid of step (a).

Within the scope of the invention are also included the steps of:

d) passing the second vapor stream to a scrubber utilizing water, wherein a third vapor stream containing uncondensed gases is removed overhead and a third liquid stream is removed from the bottoms; and

e) combining said third liquid stream with said first vapor stream prior to the indirect cooling of step (b).

This embodiment of the invention provides for the near total recovery of acrylic acid from the gaseous reactor effluent by combining the bottoms stream from the scrubber with the first vapor stream produced from the quench system.

The invention may be best understood by reference to the drawings.

## Description of the Drawings

Figure 1 shows the prior art method of obtaining an aqueous acrylic acid solution for further purification.

Figure 2 shows an embodiment of the present invention utilizing the recycle quench.

Figure 3 discloses a unique embodiment wherein all the steps necessary to obtain the aqueous acrylic acid solution are performed in one column.

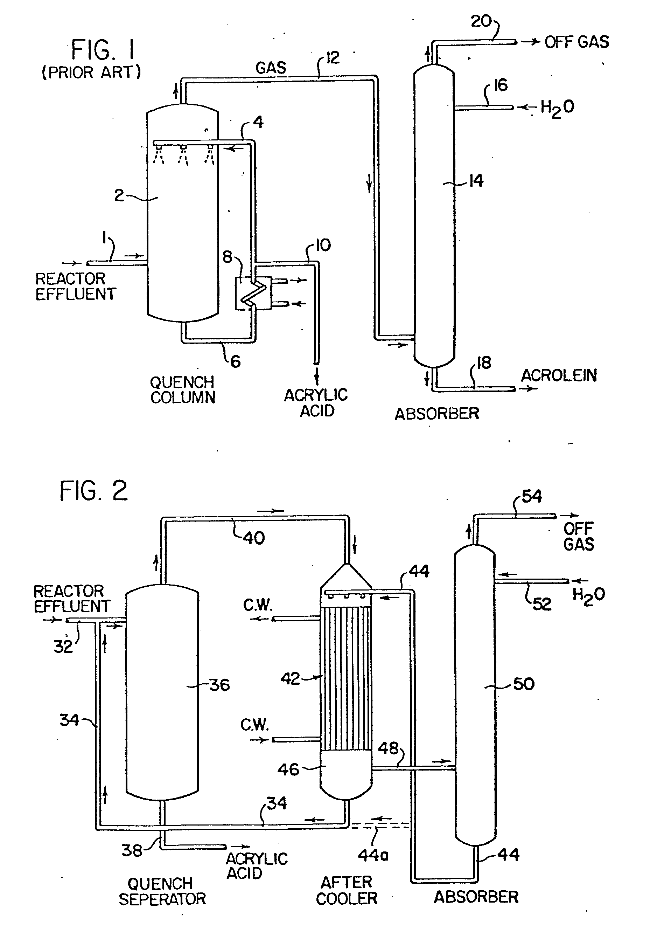

Referring to Figure 1, the gaseous reactor effluent 1 is passed to quench column 2. The purpose of this column is to cool the gaseous reactor effluent and condense the acrylic acid. Quenching liquid is provided through line 4 and is sprayed countercurrent with the reactor effluent stream. The condensed acrylic acid and water are removed from the quench column through line 6, indirectly cooled in exchanger 8, and then split into two streams. A portion is passed through line 4 as quench liquid previously described, and an aqueous solution of acrylic acid is removed through line 10 and passed to further recovery and purification (not shown). The vapor stream 12 removed from the quench column overhead typically contains acrylic acid, acrolein, inert gases and impurities. This is passed to an absorber 14. Water enters the absorber overhead through line 16 at a temperature sufficient to absorb the acrolein and thus allow a liquid stream 18 containing acrolein to be removed from the bottoms of the absorber and passed to further purification. The inert gases are removed overhead from the absorber through line 20. As can be noted from this drawing, not all of the acrylic acid is recovered in the quench column. A portion is removed in the gaseous overhead stream and exits the system with the acrolein from the bottoms of the absorber. Further, the total stream of quench liquid plus the aqueous acrylic acid product is cooled through exchanger 8, resulting in large capital investment for exchanger.

Figure 2 shows an embodiment of the present invention utilizing recycle quench. The reactor effluent is passed through line 32, combined with quenching liquid 34, and passed to quench separator 36. As shown in this figure, the quenching occurs with cocurrent contact prior to the entry into the separator. The quenching may also be countercurrent within the separator as shown in Figure 1. In the separator 36, the aqueous acrylic acid solution is removed as a bottoms stream through line 38, with the remaining vapors passing overhead through line 40. These vapors are then cooled indirectly in heat exchanger 42. Prior to their entry into the heat exchanger, a recycle stream 44 can be combined with these vapors.

The vapors are then partially condensed in exchanger 42 through the use of cooling water. The bottommost section 46, of exchanger 42, serves as a separator. The condensed liquid containing acrylic acid is passed through line 34 as the quench liquid. The vapors are sent through line 48 to scrubber 50.

The purpose of scrubber 50 is to remove the uncondensible gases and acrolein, and to recover any remaining acrylic acid. Water is passed to scrubber 50 through line 52. The inert gases containing some acrolein is removed overhead through line 54, and a liquid bottoms stream containing acrylic acid is removed through line 44. This stream can either be combined with the vapors removed from the quench system as shown by the solid line, or can be combined directly with the quench liquid in 34 as shown by the dotted line 44 a.

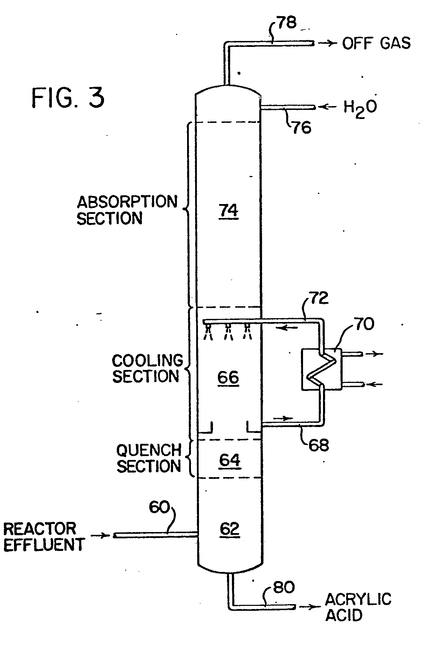

Figure 3 shows an embodiment of the invention wherein all the processing steps are combined in one column. The reactor effluent is sent through line 60 to the bottommost portion of the column 62. Above section 62 is the adiabatic section 64. Liquid falling from the cooling section above cools the reactor effluent thus condensing acrylic acid. In cooling section 66, liquid is removed from a side tray through line 68. This liquid is cooled in exchanger 70 and is recycled back to the cooling section through line 72 and sprayed countercurrent with the upflowing gases. This produces the liquid necessary for recycle and for quench.

The remaining uncondensed gases then pass through absorption section 74. Water enters the top of this section through line 76 and operates in the same manner as the absorber shown in Figure 2. The uncondensed gases with acrolein are removed overhead through line 78, and the condensed liquid falls into cooling section 66 with the condensed acrylic acid cascading down the tower. Finally, the aqueous solution of acrylic acid is removed through line 80 and passed to further recovery and purification.

Thus, this embodiment illustrates a process for the recovery of acrylic acid from a gaseous reactor effluent containing acrylic acid, acrolein, water and impurities produced from the catalytic vapor phase oxidation of propylene or acrolein in a single column having a quenching, cooling and absorbing section, comprising the steps of:

a) the reactor effluent is quenched with a quench liquid from the cooling section;

b) said quenched reactor effluent is directly cooled with a cooling medium to condense a portion of said effluent;

c) said condensed effluent is indirectly cooled prior to the direct cooling;

d) the acrylic acid is absorbed in an absorbing section by contacting the quenched, cooled reactor effluent with water and

e) an aqueous solution containing acrylic acid is removed from the bottom of said column.

The use of such a quench allows for excellent recovery of acrylic acid, improves the elimination of low boiling reaction products, and has no need for refrigeration as found in the prior art. The cooling of the reactor effluent to condense acrylic acid is accomplished by evaporation of the quench liquid. The reactor effluent thus drops in temperature to its saturation temperature at its operating pressure. The operating temperatures and pressures vary depending on whether propylene or acrolein is used as a feed. The pressure of such systems are well known in the art and usually between 0.14 and 0.70 kg/cm while the reactor effluent temperature is usually between 300 and 600°C. The adiabatic quench usually operates at temperatures above 70°C, favorably at temperatures of between 70 and 90°C.

The quenching of the reactor effluent may be cocurrent or countercurrent, with cocurrent being preferred. A shown in Figure 2, quenching may take place in an expanded portion of the reactor effluent line prior to its entry into the quench separator. The quench is sprayed cocurrently with the flow of the gases. However, quenching may also be provided countercurrently with spray nozzles at the top of the quench separator as known in the prior art.

The gases from the quench separator are then indirectly cooled in a shell and tube heat exchanger. Typically cooling water is used as a cooling medium. Because of the adiabatic nature of the quench, no refrigeration is necessary for the operation of this process. No refrigeration is required because a scrubber is used to enhance acrylic acid recovery. Other processes known in the art use a refrigerated quench section to improve recovery efficiency.

The indirect cooling achieves partial condensation of the remaining gaseous stream. The liquid resulting from this condensation, at a temperature of approximately 40°C (again depending upon the pressure) is then used as the quench liquid described previously. By obtaining the quench liquid in this manner, polymerization is greatly reduced as is the need for external cooling.

The vapors resulting from the indirect cooling can then be passed to a gas scrubber. The purpose of this scrubber is to remove uncondensible gases and acrolein from the remaining acrylic acid and water. Water is fed to the scrubber at a water/feed ratio of between 0.05 and 0.15, favorably between 0.05 and 0.10 by wt. with 0.075 being preferred. The operating temperature of this water is approximately 40°C but can range between 32°C and 45°C. At 40°C, no external refrigeration is necessary. The number of trays found in the scrubber will of course be a function of the feed composition and the amount and temperature of the water used to scrub. Typically this column consists of between 10 to 20 absorption trays, favorably 15 trays, with the feed entering below the first tray. Acrylic acid and water are recovered in the bottoms of the scrubber.

For total recovery purposes, this stream can be recycled to one of two locations. It can be combined with the vapors from the quench system prior to indirect cooling, or it can be combined directly with the quench liquid recovered from the indirect cooling that is sent to the quench system. Thus excellent recovery of acrylic acid can be obtained through using the present invention.

The aqueous acrylic acid solution recovered from the present invention is passed to further recovery and purification. Such purification is known in the art and can be found in US—PS 3,830,707, US—PS 3,859,175 and US—PS 3,433,831. These patents disclose various processes such as extraction or entrainment with various solvents to separate acrylic acid into final product purity.

## Example

A gaseous reactor effluent stream produced from the vapor phase oxidation of propylene, and containing 50% acrylic acid with the remainder being acetic acid, acrolein, water, and gases such as nitrogen and carbon dioxide, were passed to the invention's quench system. The quench system consisted of a 366 cm long quench column containing 5 spray nozzles for concurrent quenching and having a quench pot located at the bottoms of the column for separating the vapor from the liquid phase. Reactor effluent entered the quench column at a temperature of 200°C. All liquid recovered from the indirect heat exchanger, containing approximately 38% acrylic acid, was passed to the quench column spray nozzles.

After quenching, the liquid was separated from the vapors. The aqueous solution removed from the separator contained on the average, 60 wt.% acrylic acid. The quench column vapors at a temperature of 79°C were then passed to a 20.3 cm vertical shell and tube heat exchanger with a spray section above it and a separation pot below. To simulate a 3-cycle bottoms stream, water containing acrylic acid was passed to the spray section prior to the indirect heat exchange.

Cooling water was used on the shell side of the exchanger to cool the gases to a temperature of 40°C. The phases were then separated, with the quench liquid containing approximately 38% acrylic acid and the gases containing approximately 1 to 2% acrylic acid.

Without the operation of a scrubber to recover the remaining acrylic acid, the invention's quench system with the indirect exchanger allows for an 88% recovery efficiency of acrylic acid. The addition of a scrubber column will allow near complete recovery to be accomplished.

## Claims

1. Process for the recovery of acrylic acid from a gaseous reactor effluent containing acrylic acid, acrolein, water and impurities produced from the catalytic vapor phase oxidation of propylene or acrolein characterized in that
a) the gaseous reactor effluent is quenched with a quench liquid wherein a first liquid stream containing acrylic acid and a first vapor stream containing acrylic acid are formed;
b) said first vapor stream is cooled indirectly to form a second liquid stream containing acrylic acid and a second vapor stream and
c) said second liquid stream is passed as the quench liquid of step (a).

2. The process according to claim 1 characterized in that the quenching of the gaseous reactor effluent is accomplished cocurrently.

3. The process according to claim 1 or 2 characterized in that the reactor effluent is quenched to a temperature of between 70 and 90°C.

4. The process according to any of claims 1 to 3 characterized in that the feed is propylene.

5. The process according to any of claims 1 to 4 characterized in that additionally
d) said second vapor stream is passed to a scrubber utilizing water, wherein a third vapor stream containing uncondensed gases is removed overhead and a third liquid stream containing acrylic acid is removed from the bottom.

6. The process according to claim 5 characterized in that the water/feed ratio of the scrubber is between 0.05 and 0.10.

7. The process according to claim 5 or 6 characterized in that the scrubber contains between 10 to 20 absorption trays.

8. The process according to any of claims 5 to 7 characterized in that said third liquid stream is combined with said first vapor stream prior to the indirect cooling of step (b).

9. The process according to any of claims 5 to 7 characterized in that said third liquid stream is combined with said second liquid stream of step (b) and the combined streams are passed as the quench liquid of step (a).

10. Process for the recovery of acrylic acid from a gaseous reactor effluent containing acrylic acid, acrolein, water and impurities produced from the catalytic vapor phase oxidation of propylene or acrolein in a single column having a quenching, cooling and absorbing section, characterized in that

a) the reactor effluent is quenched with a quench liquid from the cooling section;

b) said quenched reactor effluent is directly cooled with a cooling medium to condense a portion of said effluent;

c) said condensed effluent is indirectly cooled prior to the direct cooling;

d) the acrylic acid is absorbed in an absorbing section by contacting the quenched, cooled reactor effluent with water and

e) an aqueous solution containing acrylic acid is removed from the bottoms of said column.

## Revendications

1. Procédé pour la récupération d'acide acrylique à partir d'un effluent gazeux de réacteur contenant de l'acide acrylique, de l'acroléine, de l'eau et des impuretés, provenant de l'oxydation catalytique en phase vapeur du propylène ou de l'acroléine, caractérisé par le fait que:

a) l'effluent gazeux du réacteur est refroidi à l'aide d'un liquide de refroidissement direct, ce qui forme un premier flux liquide contenant de l'acide acrylique et un premier flux vapeur contenant de l'acide acrylique;

b) ledit premier flux vapeur est refroidi indirectement pour former un second flux liquide contenant de l'acide acrylique et un second flux vapeur;

c) ledit second flux liquide est utilisé comme liquide de refroidissement direct pour l'étape a).

2. Procédé selon la revendication 1, caractérisé par le fait que le refroidissement direct de l'effluent gazeux du réacteur est effectué par circulations parallèles.

3. Procédé selon l'une des revendications 1 ou 2, caractérisé par le fait que l'effluent du réacteur est refroidi jusqu'à une température comprise entre 70°C et 90°C.

4. Procédé selon l'une des revendications 1 à 3, caractérisé par le fait que l'alimentation est constituée par du propylène.

5. Procédé selon l'une des revendications 1 à 4, caractérisé par le fait que, en outre:

d) ledit second flux vapeur est envoyé dans un épurateur utilisant de l'eau, d'où on extrait en tête un troisième flux vapeur, contenant des gaz non condensés et d'où on extrait à la base un troisième flux liquide contenant de l'acide acrylique.

6. Procédé selon la revendication 5, caractérisé par le fait que le rapport eau/alimentation de l'épurateur est compris entre 0,05 et 0,10.

7. Procédé selon l'une des revendications 5 ou 6, caractérisé par le fait que l'épurateur contient entre 10 et 20 plateaux d'absorption.

8. Procédé selon l'une des revendications 5 à 7, caractérisé par le fait que ledit troisième flux liquide est combiné avec ledit premier flux vapeur avant le refroidissement indirect de l'étape b).

9. Procédé selon l'une des revendications 5 à 7, caractérisé par le fait que le troisième flux liquide est combiné avec le second flux liquide de l'étape b) et que les flux combinés sont utilisés comme liquide de refroidissement direct dans l'étape a).

10. Procédé pour la récupération d'acide acrylique dans un effluent gazeux de réacteur contenant de l'acide acrylique, de l'acroléine, de l'eau et des impuretés, provenant de l'oxydation catalytique en phase vapeur du propylène ou de l'acroléine, dans une colonne unique comportant une section de refroidissement direct, une section de refroidissement indirect et une section d'absorption, caractérisé par le fait que:

a) l'effluent du réacteur subit le refroidissement direct à l'aide d'un liquide de refroidissement provenant de la section de refroidissement;

b) ledit effluent de réacteur, après l'opération de refroidissement direct, est directement refroidi par un fluide de refroidissement, afin de condenser une partie dudit effluent;

c) ledit effluent condensé est refroidi indirectement avant le refroidissement direct;

d) l'acide acrylique est absorbé dans une section d'absorption par contact de l'effluent du réacteur ayant subi les opérations de refroidissement direct et indirect avec de l'eau; et

e) une solution aqueuse contenant de l'acide acrylique est extraite à la base de la colonne.

## Patentansprüche

1. Verfahren zur Gewinnung von Acrylsäure aus einem gasförmigen Reaktorabstrom, der Acrylsäure, Acrolein, Wasser und Verunreinigungen enthält, wie er bei der katalytischen Gasphasenoxidation von Propylen oder Acrolein gebildet wird, dadurch gekennzeichnet, daß

a) der gasförmige Reaktorabstrom mit einer Abschreckflüssigkeit, abgeschreckt wird, wobei ein erster Flüssigkeitssstrom, der Acrylsäure enthält, und ein erster Dampfstrom, der Acrylsäure enthält, gebildet werden;

b) der erste Dampfstrom indirekt gekühlt wird unter Bildung eines zweiten Flüssigkeitsstromes, der Acrylsäure enthält, und eines zweiten Dampfstromes; und

c) der zweite Flüssigkeitsstrom als Abschreckflüssigkeit in die Stufe (a) eingeführt wird.

2. Verfahren nach Anspruch 1, dadurch gekennzeichnet, daß das Abschrecken des gasförmigen Reaktorabstroms im Gleichstrom bewirkt wird.

3. Verfahren nach Anspruch 1 oder 2, dadurch gekennzeichnet, daß der Reaktorabstrom auf eine Temperatur zwischen 70 und 90°C abgeschreckt wird.

4. Verfahren nach einem der Ansprüche 1 bis 3, dadurch gekennzeichnet, daß als Ausgangsmaterial Propylen verwendet wird.

5. Verfahren nach einem der Ansprüche 1 bis 4, dadurch gekennzeichnet, daß zusätzlich

d) der zweite Dampfstrom in einen Gaswäscher eingeführt wird, in dem Wasser verdendet wird, wobei ein dritter Dampfstrom, der nicht-kondensierte Gase enthält, überkopf abgezogen und ein dritter Flüssigkeitsstrom, der Acrylsäure enthält, aus dem Boden abgezogen werden.

6. Verfahren nach Anspruch 5, dadurch gekennzeichnet, daß das Wasser/Beschickungs-Verhältnis des Gaswäschers zwischen 0,05 und 0,10 liegt.

7. Verfahren nach Anspruch 5 oder 6, dadurch gekennzeichnet, daß der Gaswäscher 10 bis 20 theoretische Absorptionsböden enthält.

8. Verfahren nach einem der Ansprüche 5 bis 7, dadurch gekennzeichnet, daß der dritte Flüssigkeitsstrom vor dem indirekten Kühlen in der Stufe (b) mit dem ersten Dampfstrom kombiniert wird.

9. Verfahren nach einem der Ansprüche 5 bis 7, dadurch gekennzeichnet, daß der dritte Flüssigkeitsstrom mit dem zweiten Flüssigkeitsstrom der Stufe (b) kombiniert wird und daß die kombinierten Ströme als Abschreckflüssigkeit in die Stufe (a) eingeführt werden.

10. Verfahren zur Gewinnung von Acrylsäure aus einem gasförmigen Reaktorabstrom, der Acrylsäure, Acrolein, Wasser und Verunreinigungen enthält, wie er bei der katalytischen Gasphasenoxidation von Propylen oder Acrolein in einer einzigen Kolonne mit einem Abschreck-, Kühl- und Absorptionsabschnitt gebildet wird, dadurch gekennzeichnet, daß

a) der Reaktorabstrom mit einer Abschreckflüssigkeit aus dem Kühlabschnitt abgeschreckt wird;

b) der abgeschreckte Reaktorabstrom direkt mit einem Kühlmedium gekühlt wird, um einen Teil des Abstroms zu kondensieren;

c) der kondensierte Abstrom vor dem direkten Kühlen indirekt gekühlt wird;

d) die Acrylsäure in einem Absorptionsabschnitt absorbiert wird, indem man den abgeschreckten, gekühlten Reaktorabstrom mit Wasser in Kontakt bringt; und

e) eine wäßrige Lösung, die Acrylsäure enthält, aus dem Boden der Kolonne abzieht.

FIG. I
(PRIOR ART)

GAS 12

20 → OFF GAS

4

16 ← H₂O

2

14

REACTOR
EFFLUENT

8

10

6

18 → ACROLEIN

QUENCH
COLUMN

ACRYLIC
ACID

ABSORBER

FIG. 2

54 → OFF
GAS

40

44

REACTOR
EFFLUENT

C.W.

52 ← H₂O

32

36

42

50

34

C.W.

48

46

34

38 → ACRYLIC
ACID

44a

44

QUENCH
SEPERATOR

AFTER
COOLER

ABSORBER

FIG. 3

ABSORPTION SECTION — 74

78 → OFF GAS

76 ← H₂O

72

70

COOLING SECTION — 66

68

QUENCH SECTION — 64

60

REACTOR EFFLUENT → 62

80 → ACRYLIC ACID